# EUROPEAN PATENT APPLICATION

(11) **EP 4 212 171 A1**
(43) Date of publication of application: **19.07.2023**
(21) Application number: 21857754.2
(22) Date of filing: 20.08.2021
(51) Int. Cl.: A61K 38/45, C12N 9/12, A61P 35/00

(54) **APPLICATION OF RDR PROTEIN IN TUMOR THERAPY**

(30) Priority: 21.08.2020 CN 202010848770
(71) Applicant: Peking University, Beijing 100871 (CN)
(72) Inventor: DU, Peng, Beijing 100871 (CN); QI, Ye, Beijing 100871 (CN)
(74) Representative: Ipey
(86) International application number: PCT/CN2021/113702
(87) International publication number: WO 2022/037666

(57) **Abstract**

The present disclosure provides an application of RNA-dependent RNA polymerase (RDR) in treatment of cell proliferative diseases. The RDR can be widely used for inhibiting cell proliferation, apoptosis and migration of various tumors, thereby providing a new choice for clinical treatment of tumors.

## Description

### Related applications

The present application claims the benefits of and priority to Chinese application No. 202010848770.7 filed on Aug 21, 2020. The application No. 202010848770.7 is incorporated herein by reference in its entirety.

### Field

The present disclosure is related to the field of molecular biology and tumor therapy. Specifically, the present disclosure is related to the anti-tumor use of RDR protein and its coding gene, the applications thereof as a medicament for the treatment or the adjuvant treatment of tumors.

### Background

Malignant tumor (cancer) is a disease caused by typical abnormal cell proliferation, and has evolved as the greatest threat to human health. More than 100 types of cancer have been known to influence humans, with approximately 14.1 million new cases each year (excluding skin cancers other than melanoma), which leads to approximately 8.8 million deaths (accounting for 15.7% of overall death toll). At present, therapies which have been tried to cure malignant tumors mainly include surgery, chemotherapy, radiotherapy, hormone therapy and targeted therapy, etc. However, a critical characteristic of multiple cancers is chemosensitivity (e.g., non-small cell lung cancer, pancreatic cancer) followed by the rapid emergence of chemoresistance. National Cancer Institute has defined a number of refractory cancers as having high mortality rate and lacking treatment options, to highlight the demand for novel treatment options. Apart from the inability of overcoming drug resistance to existing therapies, many chemotherapeutic agents and targeted medicines often have characteristics including having severe side effects and targeting single pathway, which have greatly limited their clinical application.

### Summary

The inventors have surprisingly discovered that RNA-dependent RNA polymerase (RDR) is able to inhibit excessive cell proliferation, especially tumor cell proliferation and growth, so that it can be used as an effective therapy for tumors.

Specifically, the first aspect of the present disclosure provides the use of RDR or the functional variants thereof in preparing medications for the treatment or prevention of cell proliferative diseases.

In one embodiment, the cell proliferative disease is tumor, and more particularly, the tumor can be benign or malignant tumor (or cancer), and preferably, the malignant tumor includes solid tumor, lymphoma or hematologic tumor, e.g., leukaemia, breast cancer, skin cancer, osteocarcinoma, liver cancer, brain cancer, laryngeal cancer, gallbladder cancer, pancreatic cancer, rectal cancer, parathyroid gland cancer, stomach cancer, bronchogenic cancer, kidney cancer, basal cell carcinoma, ulcer, and papillary squamous cell carcinoma, sarcoma, myeloma, giant cell tumor, small cell lung cancer, non-small cell lung cancer, islet cell tumor, primary brain tumor, acute or chronic lymphocytoma or granulocyte tumor, hair cell tumor, adenoma, medullary carcinoma, pheochromocytoma, mucosal nerve cell carcinoma, intestinal ganglloneuromas, proliferative corneal nerve tumor, spermatocytoma, liomyoma, cervical cancer, colorectal cancer, neuroblastoma, retinoblastoma, rhabdomyosarcoma, malignant hypercalcemia, glioblastoma multiforme, melanoma or epiermoid carcinoma.

In another embodiment, the RDR is selected from the group consisting of RDR1, RDR2 and RDR6, preferably the RDR is RDR1; even more preferably, the RDR is derived from a plant, such as, monocotyledonous or dicotyledonous plant, preferably the plant is selected from the group consisting of *Arabidopsis thaliana,* rice, tobacco, *Populus trichocarpa*, soybean, grape, tomato, potato, barley, slender false brome, maize, sorghum, cacao, rice, petunia, cotton and cucumber.

In another embodiment, the RDR is the nucleic acid encoding RDR protein, such as, genomic DNA, cDNA, or CDS, preferably, the nucleic acid is integrated in a vector, such as, a plasmid vector or a viral vector, preferably, the viral vector is selected from the group consisting of retroviral vector, lentiviral vector, and adenovirus vector; or the functional variant is a truncated RDR retaining the RDRp domain. For example, the functional variant is the RDRp domain.

The second aspect of the present disclosure provides a pharmaceutical composition for the treatment or prevention of cell proliferative diseases, comprising RDR, and a pharmaceutically acceptable carrier.

The third aspect of the present disclosure provides a method for suppressing cell proliferation, comprising *in vitro* introduction of the RDR into a target cell, wherein the target cell is a normal cell, or a cell with abnormal proliferation regulation, such as, a tumor cell.

The fourth aspect of the present disclosure provides a method for inhibiting tumor growth and/or metastasis, comprising the step of administrating a therapeutically effective amount of RDR protein, the nucleic acid encoding RDR protein, or the pharmaceutical composition described by the second aspect above to a subject in need thereof.

In one embodiment, the present disclosure provides the use of RDR protein in preparing a reagent/medication/composition/kit for inhibiting tumor growth and/or metastasis.

The fifth aspect of the present disclosure provides a method for improving the stability or increasing the expression of miRNAs in cells, comprising the step of contacting RDR with a target cell, or introducing RDR into a target cell. Preferably, the target cell is a normal cell, or a cell with abnormal proliferation regulation, such as, a tumor cell or a cancer cell.

In one embodiment, the present disclosure provides the use of RDR protein in preparing a reagent/medication/composition/kit for improving the stability or increasing the expression of miRNAs in animal cells. Preferably, the target cell is a normal cell, or a cell with abnormal proliferation regulation, such as, a tumor cell or a cancer cell.

The seventh aspect of the present disclosure provides a method for regulating cell cycle, comprising the step of contacting RDR with a target cell, or introducing RDR into a target cell. Preferably, the target cell is a normal cell, or a cell with abnormal proliferation regulation, such as, a tumor cell or a cancer cell.

In one embodiment, the present disclosure provides the use of RDR protein in preparing a reagent/medication/composition/kit for inhibiting tumor growth and/or metastasis.

The eighth aspect of the present disclosure provides a method for increasing the expression of the low-expression miRNAs in tumor cells, comprising the step of contacting RDR with a tumor cell, or introducing RDR into a tumor cell.

In one embodiment, the present disclosure provides the use of RDR protein in preparing a reagent/medication/composition/kit for increasing the expression of the low-expression miRNAs in tumor cells.

The ninth aspect of the present disclosure provides a method for reducing the expression of cell cycle-related genes in tumor cells, comprising the step of contacting RDR with a tumor cell, or introducing RDR into a tumor cell.

In one embodiment, the present disclosure provides the use of RDR protein in preparing a reagent/medication/composition/kit for reducing the expression of cell cycle-releted genes in tumor cells.

According to any of the previous aspects, the RDR is selected from the group consisting of RDR1, RDR2 and RDR6, preferably the RDR is RDR1; even more preferably, the RDR is derived from a plant, preferably the plant is selected from the group consisiting of *Arabidopsis thaliana,* rice, tobacco, *Populus trichocarpa,* soybean, grape, tomato, potato, barley, slender false brome, maize, sorghum, cacao, rice, petunia, cotton and cucumber.

According to any of the previous aspects, the RDR is a functional RDR protein or a nucleic acid molecule encoding RDR protein.

According to any of the previous aspects, the subject or cell is preferably or is preferably derived from human or animals. For example, the animals are non-human mammals, such as cattle, swine, horses, chickens, cats or dogs, etc.

The RDR protein provided in the present disclosure (for example, the plant RDR1) is capable of effectively suppressing cancer cells. The mechanism of action is as follows: RDR protein is able to up-regulate a broad spectrum of miRNAs which have reduced expression in cancer cells by adding tails at the terminus of these miRNAs, while in normal cells (tissues), the feedback mechanism will maintain the miRNA expression level so that overexpression of RDR will not result in proliferation inhibition or up-regulation of miRNAs, thereby reducing the possible side effects in clinical application. Compared to conventional chemical drugs, RDR posesses better specificity for the tumor cells and is capable to inhibit multiple cancers broadly.

Furthermore, it has also been verified in the present disclosure that RDR is not working on single target, but is able to regulate the expression of a plurality of genes. As shown by the RNA-seq data for various cancer cells, 60-70 genes were simultaneously down-regulated significantly, which involve distinct pathways such as cell proliferation and focal adhesion (related to cancer invasion), as revealed by GSEA analysis, indicating this potential therapy will hardly produce drug resistance caused by a few gene mutations arising in cancer cells, making it extremely promising for clinical applications.

### Brief description of the drawings

Figure 1 shows the results of bioinformatics analysis of the RDR1 gene sequence. The sequence of the key domain RdRp is highly conserved among the RDR1s of different species.
Figure 2 is a flow chart showing the experiment for over-expression of RDR1 in cancer cells.
Figure 3 shows the expression of RDR1 in cancer cells as detected by Western Blot. After induction by Dox at a high concentration (4 µg/mL) for 3 days, elevated expression of Flag-OsRDR1/AtRDR1 was detected across all cell lines as detected by Western Blot, while the background expression of RDR1 can hardly be detected in control cells without Dox induction.
Figure 4 shows the proliferation of cancer cells after RDR1 expression was induced by Dox. Compared with control cells without induction by Dox, significant inhibition of cancer cell growth was consistently observed after addition of Dox to stably include the expression of RDR1, from Day 4 to Day 6, when the experiment ended, while the growth rate and number of normal cells remained basically unchanged.
Figure 5 shows the results of the cell invasion assay of the OsRDR1/AtRDR1 stable cell line. After induced by Dox at a high concentration for 5 consecutive days and transfered to a chamber and kept for 24 hours, it can be seen that induction of RDR1 greatly reduced the amount of cancer cells that had successfully went through the chamber.
Figure 6 shows the apoptosis results with the OsRDR1/AtRDR1 stable expression cell line. Compared with the cancer cell line HepG2, H1299 and the normal cell line NIH/3T3, the proportion of apoptotic cells in each experiment group was within normal range, i.e., no change in apoptosis was observed in the OsRDR1/AtRDR1 stable expression cell line after induction.
Figure 7 shows a schematic diagram of a RDR1 mutant and its expression in cancer cells. Figure 7A shows three potential active sites identified by conserved domain analysis are mutated separately (D429A, D431A, D433A). Figure 7B shows the expression of the mutated OsRDR1 and the wild-type control protein as detected by Western Blot, with no significant difference identified.
Figure 8 shows the proliferation of cancer cells after RDR1-mut was induced by Dox. The result indicates OsRDR1-Mut cancer cells treated with Dox at a high concentration had no effect on the cell proliferation.
Figure 9 shows a schematic diagram of truncated RDR1 (Figure 9A), the cellular expression of truncated RDR1 (Figure 9B), and the impact on the proliferation of cancer cells (Figure 9C).
Figure 10 shows the mouse A549LUC-OsRDR1 tumor-bearing model experiment. Figures 10A, B and C show over-expression of RDR1 can significantly reduce the volume and weight of the tumors; Figure 10D shows that RDR1 reduces the intensity of luciferase expression in the A549 tumor.
Figure 11 shows the RNA-seq analysis results of a RDR1 stable expression cell line.
Figure 12 shows the GSEA cell cycle analysis results of a RDR1 stable expression cell line.

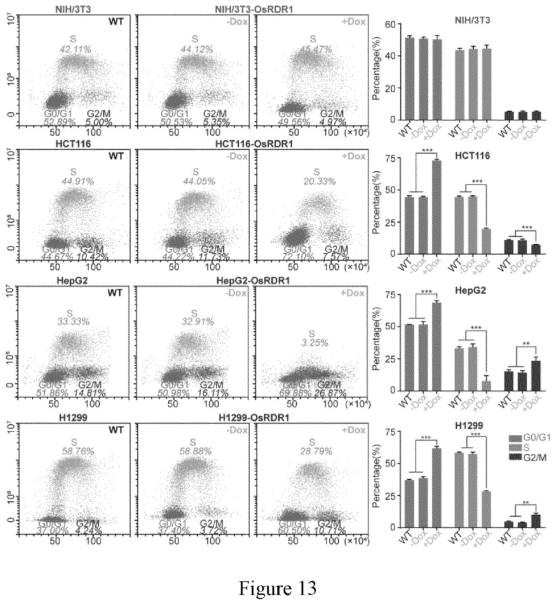

Figure 13 shows the cell cycle experiments with an OsRDR1 stable expression cell line. As compared to wild-type cells (without virus transfection) and cell lines without induction by Dox (no RDR1 over-expression), after overexpression of RDR1 protein in cancer cells for 6 days, most cells were arrested in G1 phase, while the proportion of cells in S phase cells reduced significantly and some were arrested in G2 phase to some extent.
Figure 14 shows the possible target genes of RDR1 identified in RNA-seq analysis and relative verification. Figure 14A shows the types of genes regulated by RDR1, among which the cell cycle-related genes were most abundant; Figure 14B shows the proto-oncogenes annotated by GO analysis. These proto-oncogenes have two major functions, i.e., cell cycle regulation and cell population proliferation regulation; Figure 14C shows the expression of marker genes in cancer cells as detected by Western Blot.
Figure 15 shows that RDR1 protein can restore the expression of miRNAs which had reduced expression in cancer cells and miRNA-target gene interaction network.
Figure 16 shows RDR1 protein was able to up-regulate the expression of miRNAs with specific tailing mechanism.
Figure 17 shows the cellular localization of RDR1 protein.

### Detailed description

The present disclosure can be further understood by the following examples, however, it should be understood that the purpose of these examples is not to limit the invention. Modifications of the invention now known or further developed are considered to fall within the scope of the invention described herein and claimed below.

### RDR

RDR stands for RNA-dependent RNA polymerase (RDR). In 1963, researchers f an enzyme that could synthesize RNA using RNA as a template in bacteriophage. It was considered as a RNA-dependent RNA polymerase expressed by bacteriophage and used for synthesis of the genomic RNA of bacteriophage. Until 1971, eukaryotic RDR was first reported in Chinese cabbage. So far, RDRs have been found in RNA viruses, plants, fungi, protozoa and lower animals, but not in higher animals, including *Drosophila,* mice and humans.

One of the most fundamental features of RDR is to catalyze the replication of RNA using RNA template. In RNA viruses which contain no DNA, RDR is an essential encoded protein. RDR-mediated RNA replication can be initiated at or near the 3'-terminus of the RNA template via primer-independent (*de novo* synthesis) or primer-dependent mechanisms. Through the continuous addition of NTP (nucleoside triphosphate, NTP) to the 3'-OH end, synthesis of the complementary strand is accomplished so as to convert single-stranded RNA into double-stranded RNA. More importantly, an indispensable step which lies upstream in the siRNA synthesis pathway is just the RDR-mediated double-stranded synthesis. Therefore, RDR is a necessity and must in species possessing siRNA pathway. In general, RDR regulates development, maintains genome integrity and produces antiviral immunity by mediating the production and amplification of siRNAs.

From evolution perspective, there are three RDR copies in eukaryotic ancestors: RDRα, RDRβ and RDRy, wherein RDRβ notebly diverges from RDRy: plants merely have RDRy, whereas protists and invertebrates only contain RDRβ. Taking plants as an example, in *Arabidopsis thaliana,* a model plant, three RDR members with determined function, i.e., AtRDR1, AtRDR2 and AtRDR6, are reported, all of which belong to the RDRα family. While the plant-specific RDRγ in *Arabidopsis thaliana* correspond to AtRDR3, AtRDR4 and AtRDR5, whose biological functions have not been clearly identified. Meanwhile, as compared with the dicotyledonous model plant *Arabidopsis thaliana,* rice, a representative monocotyledonous plant, contains five members from RDR family as well, including OsRDR1, OsRDR2 and OsRDR6 out of RDRα, and OsRDR3a and OsRDR3b out of RDRy, the plant-specific branch.

There are two major biochemical functions of plant RDRs: RNA polymerase and terminal transferase, both of which have very conserved domains. For example, RDR1, RDR2 and RDR6 out of RDRα branch in *Arabidopsis thaliana* have a typical DLDGD catalytic motif at the C-terminus, and the same motif is also found in RDRs in many other plants. The function of these RDRs was initially associated with anti-virus, then RDR2 and RDR6 were found to involve in the regulation of plant chromosome structure and endogenous tasiRNA pathways as well. Taking RDR1 for example, RDR1 is one of the first gene silencing pathway members discovered in plants, and the first reported gene silencing pathway gene to have antiviral activity. Through small RNA sequencing analysis, RDR1 is demonstrated to be directly involved in the viral siRNA synthesis. In addition, RDR1 can recognize uncapped mRNA and synthesize double-stranded RNA using this mRNA as template to initiate siRNA pathway of *Arabidopsis thaliana,* reducing the fundamental plant physiological activities as well as the energy and materials supply required by virus replication, thereby containing virus replication and spread. However, there has no report on the relationship between RDR and tumor.

More specifically, structural analysis indicates (Poch O, Sauvaget I, Delarue M, et al. Identification of four conserved motifs among the RNA-dependent polymerase encoding elements [J]. The EMBO journal, 1989, 8(12): 3867-3874.): RDR possesses a key domain, RDRp, which has a right-handed stereoscopic conformation and comprises three subdomains: One finger subdomain, One palm subdomain and One thumb subdomain. The palm subdomain is highly conserved among all RDRs,. In RdRp, the palm subdomain has two conserved aspartic acid residues that can interact with two divalent metal ions, thereby promoting nucleophilic attack and facilitating the synthesis of new RNA strands. Moreover, RDRp has six conserved structural motifs (A-F), most of which are located within palm subdomain (A-E), while the F motif is located in finger subdomain. all of the motifs have been verified to influence the process of correct incorporation and recombination of nucleotides.

Bioinformatics analysis, such as sequence alignment, showed that the eukaryotic RDRp domains share a common conserved functional motif site "DXDGD" (X stands for any amino acid) (such as "DLGDD" in plants, as described above). Through the conservation analysis of the RDR amino acid sequences from 37 different fungal species, the non-catalytic N-terminus of the RDRs across species containing the above motif were found to have an extremely low conservation score. Introducing a mutation within the DYDGD motif (aspartic acid to alanine at position 867) to AtRDR6 completely abolished the RNA polymerase activity using ssRNA as template and the template-independent 3'-terminal nucleotidyl transferase activity of RDR6 (Curaba J, Chen X. Biochemical activities of Arabidopsis thaliana RNA-dependent RNA polymerase 6 [J]. Journal of Biological Chemistry, 2008, 283(6): 3059-3066).

As shown above, all of the members in RDR family contain RDRp sequences that are highly similar in structure and function. The examples of the present disclosure demonstrates even a single RDRp sequence is able to produce effects comparable to those of the full-length RDR1, as shown below. Therefore, those skilled in the art will anticipate all members of the RDR family, not limited to RDR1 in the examples, should produce consistent technical effects when used in the present disclosure.

Unless otherwise specified, "RDR", when used separately in the present disclosure, could refer to the RDR protein, or the coding gene or nucleotide sequence encoding RDR protein. The RDR gene or RDR protein of the present disclosure can be derived from any species, as long as the species contains the RDR gene as described above. For example, the RDRs described in the present disclosure can be derived from plants, more specifically, monocotyledonous or dicotyledonous plants, such as *Arabidopsis thaliana,* rice, tobacco, *Populus trichocarpa,* soybean, grape, tomato, potato, barley, slender false brome, maize, sorghum, cacao, rice, petunia, cotton or cucumber etc. Alternatively, the RDR has at least 60%, at least 70%, at least 80%, at least 85%, at least 90%, at least 95% similarity, e.g., 80%, 81%, 82%, 83%, 84%, 85%, 86%, 87%, 88%, 89%, 90%, 91%, 92%, 93%, 94%, 95%, 96%, 97%, 98%, or 99% similarity to the RDRs from the above-mentioned plants such as *Arabidopsis thaliana* or rice, and has the same functions.

The RDRs described in the present disclosure can be RDR1, RDR2, RDR3, RDR4, RDR5 or RDR6. RDR1-RDR6 comprise a part of subfamily members, such as RDR1a and RDR1b in *Selaginella tamariscina.* In addition, The RDRs further comprise QDE-1 from *Neurospora crassa,* Rdp-1 from *Schizosaccharomyces* and EGO-1 from *Caenorhabditis elegans*, which have been clearly biochemically characterized from cells. Notably, RDR6 from *Arabidopsis thaliana* is also named SGS2 and SDE1, which falls within the scope of the present disclosure as well.

The functional variants of RDR can also be used in the present application. The functional variant refers to the RDR sequences that have or substantially have the same or similar functions as the wild-type RDRs. In some embodiments, the RDR comprises the functional variants of RDR. The variants can be heterogenous polypeptides obtained by fusion of intact or part of C-terminus, N-terminus or internal fragment of the RDR. In some embodiments, the functional variant of RDR is a truncated RDR or a fusion of the truncated RDR that retains the RDRp domain. In other embodiments, the variant of RDR merely comprises or consists of the RDRp domain.

In some embodiments, the functional variants of RDR can be prepared by modifying the RDR sequence by substitutions, additions or deletions to obatin functionally equivalent molecules.

Due to the degeneracy of the nucleotide encoding sequence, other DNA sequences encoding the amino acid sequences that are substantially the same as those encoded by the RDR genes can be used in practice. The variant of RDR of the present disclosure includes, but not limited to, those containing whole or a part of the amino acid sequence of the RDR protein as the primary amino acid sequence, including the modified sequences in which functionally equivalent amino acid residues are substituted. One or more amino acid residues within the sequence can be substituted with another amino acid of similar polarity, which acts as a functional equivalent, resulting in a silent modification. The substitute for the amino acid within the sequence can be selected from other members of the same class as the amino acid. For example, non-polar (hydrophobic) amino acids include alanine, leucine, isoleucine, valine, proline, phenylalanine, tryptophan and methionine. Polar neutral amino acids include glycine, serine, threonine, cysteine, tyrosine, asparagine and glutamine. Positively charged (basic) amino acids include arginine, lysine and histidine. Negatively charged (acidic) amino acids include aspartic acid and glutamic acid. The variant may comprise a signal sequence (or a leader sequence) at the N-terminus of the protein that directs the transfer of the protein during translation or post-translation. The polypeptide can also be conjugated with a linker or another sequence to simplify synthesis, purification or characterization of the polypeptide (e.g., poly-His), or to enhance binding between the polypeptide and a solid support. For example, the polypeptide can be conjugated with the Fc region of an immunoglobulin.

### Pharmaceutical compositions and treatments

The pharmaceutical composition of the present disclosure may comprise only the RDR protein of the present disclosure or the nucleic acid molecule encoding the RDR protein, or may further comprise a pharmaceutically acceptable carrier for the administration in a patient.

"Carrier" refers to a diluent, adjuvant, excipient, or carrier with which a therapeutic agent is administered. Such pharmaceutical carriers can be sterile liquids, such as water and oils, including those of petroleum, animal, vegetable or synthetic origin, for example, peanut oil, soybean oil, mineral oil, sesame oil, olive oil, gels (e.g., hydrogels), etc. Saline is the preferred carrier when the pharmaceutical composition is administered intravenously. Saline solutions and aqueous dextrose and glycerol solutions can also be employed as a liquid carrier, particularly for the injectable solution.

Suitable pharmaceutical excipients include starch, glucose, lactose, sucrose, gelatin, malt, rice, flour, chalk, silica gel, sodium stearate, glycerol monostearate, talc, sodium chloride, skim milk, glycerin, propylene, glycol, water, ethanol, etc. If required, the composition may also contain a minor amount of a wetting agent or an emulsifier, or a pH buffering agent. These compositions can be adopted in the form of solution, suspension, emulsion, tablet, pill, capsule, powder, sustained release formulation and the like. Oral formulations may comprise a standard carrier, for example, pharmaceutical grade mannitol, lactose, starch, magnesium stearate, sodium saccharin, cellulose, magnesium carbonate, and the like.

The method of treating, preventing or containing tumors with RDR, and pharmaceutical composition comprising RDR provided by the present disclosure will be described in detail below. RDR1s, which come from common plants are specifically used as the representatives of RDR. While it can be understood by those skilled in the rt that, as mentioned above, RDR1 from other species and other RDRs out of RDR family are also applicable, and are considered to be within the scope of the invention.

### Example 1. Preparation of the cancer cell line overexpressing Arabidopsis thaliana or rice RDR1 protein

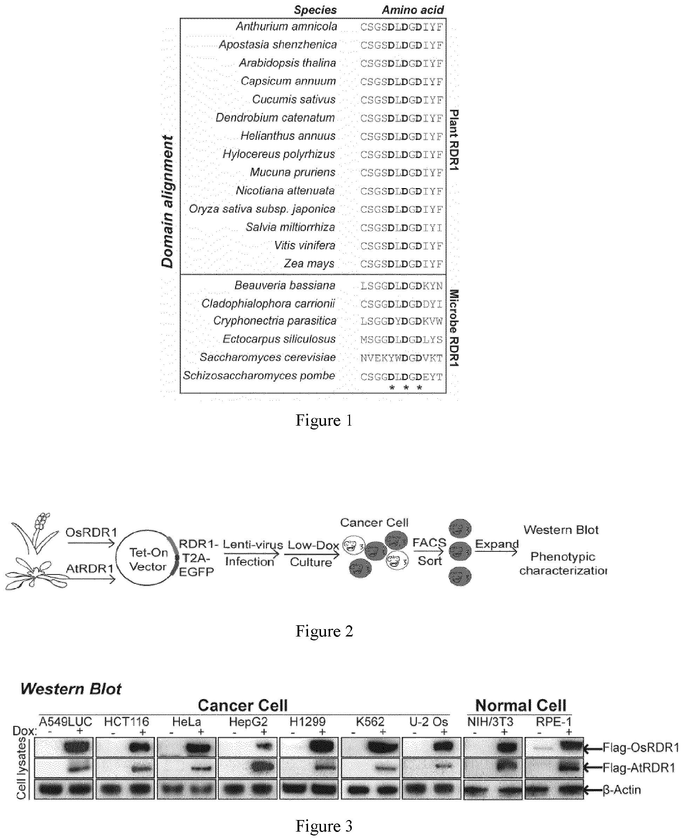

First, the sequence of RDR1 was subjected to bioinformatics analysis. By performing a BLAST search on the RDR1 sequence of *Arabidopsis thaliana* and comparing with the RDR1 sequences of 14 plants and 6 microorganisms, it was found that although various species have vast difference in morphology, the RNA-dependent RNA polymerase domains (RDRp) in the RDR1s had highly similar sequences and motifs (indicated by *), suggesting the existence of a great functional similarity among the RDR1s from different species (Figure 1).

In order to overexpress the plant RDR1 (RNA-dependent RNA polymerase 1) protein in different cancer cells, the RDR1 gene was first cloned from the model plant *Arabidopsis thaliana* and Nipponbare rice. cDNA (complementary DNA) was obtained by extraction of the genomic RNA of *Arabidopsis thaliana* and Nipponbare rice and reverse transcription using SuperScript IV. Subsequently, AtRDR1 (*Arabidopsis thaliana* RDR1) was obtained by amplification using the cDNA as a template and using AtRDR1_Sal1_F primer (SEQ ID NO: 8) and AtRDR1_BamH1_R primer (SEQ ID No: 9). Likewise, OsRDR1 was obtained by amplification using the cDNA as a template and using OsRDR1_Sal1_F primer (SEQ ID NO: 10) and OsRDR1_BamH1_R primer (SEQ ID No: 11). The above two PCR fragments were recovered and purified. The purified AtRDR1 and T2A-EGFP fragment were then ligated to a lentiviral vector using plenti_AtRDR1_CDS_F primer (SEQ ID NO: 12), plenti_AtRDR1_CDS_R primer (SEQ ID No: 13) and plenti_AtRDR1_T2A-EGFP_F primer (SEQ ID NO: 14), plenti_AtRDR1_T2A-EGFP_R primer (SEQ ID NO: 15), before confirmed by sequencing. Similarly, the purified OsRDR1 (Oryza Sativa. RDR1) and the T2A-EGFP fragment were ligated to a lentiviral vector using plenti_OsRDR1_CDS F primer (SEQ ID NO: 16), plenti_OsRDR1_CDS R primer (SEQ ID No: 17) and plenti_OsRDR1_T2A-EGFP F primer (SEQ ID NO: 18), plenti_OsRDR1_T2A-EGFP_R primer (SEQ ID No: 19).

Subsequently, 293T cells were prepared and passaged to P2 for virus packaging experiment. Using the transfection reagent Lipofectamine 3000, tube A (500 µL OptiMEM + 31 µL lipo3000) and tube B (500 µL OptiMEM + 40 µL P3000 + pOs/AtRDR1-T2A-EGFP 10 µg + pMD2G 2.5 µg + pSPAX 27.5 µg) were prepared according to the instruction, mixed and placed at room temperature for 15 min, before slowly poured into 10 cm 293T cells (density of 90%) along the wall of the dish, and mixed to homogenity. Six hours later, it was replaced by normal culture medium. After 48 hours, the virus supernatant was harvested, centrifuged at 1500 rpm for 5 minutes to remove any cell debris, aliquoted, and stored at -80 °C. Similarly, another helper virus, TetOn-3G, was packaged.

Subsequently, virus OsRDR1/AtRDR1 and TetOn-3G were mixed at a ratio of 1:1, to which 6 µg/mL polybrene was added to infect the target cells. After 12 hours of infection, it was replaced by normal culture medium containing low concentration of Dox (100 ng/mL), and the EGFP-positive cells were flow-sorted 24 hours after induction (Figure 2). When the positive cells to be sorted were expanded to a certain scale, high concentration of Dox (4 µg/mL) was added to induce RDR1 expression for about 72 hours, and then the cells were harvested and treated with RIPA cell lysis solution and PMSF. After centrifugation, the supernatant was collected and detected by Western Blot for the expression of the target protein RDR1 in cancer cells (Figure 3).

As shown in Figure 3, different cancer cell lines (including non-small cell lung cancer A549LUC, H1299, colorectal cancer HCT116, cervical cancer HeLa, liver cancer HepG2, chronic myeloid leukemia K562, osteosarcoma U2-OS) and healthy cell lines (mouse embryonic fibroblasts NIH/3T3, human retinal pigment epithelial cells RPE-1) were obtained by FACS after infection by lentivirus, which could significantly overexpress the RDR1 protein of plants after induction with high concentration of Dox. This has demonstrated that cell lines capable of producing rice RDR1 (OsRDR1) and *Arabidopsis thaliana* RDR1 (AtRDR1) , respectively, through induction by Dox, have been successfully prepared.

Among them, the gene sequence of OsRDR1 is as follows:

ATGATGGATTGGTTTATGTCTACAGTGTATTTTTATGGACCTGAGATTAATGTCTC
CAATCGTGTGGTGCGGAATTTTTCTTCTGACATAGAGAATTTCCTTCGCATTTCA
TTTGTTGACGAGGACTGCGAGAAGCTCCGTGCAACTGATTTATCCCCCCGTTCT
GCTTCAGGACATGATGCAAACAGAACTGCTCTGTATAAGAGAGTTCTGTCAGTC
CTTTCAGATGGCATCACTATTGGTGGCAAGAACTTTGAGTTTCTTGCTTTTTCTT
CAAGCCAGCTCCGAGATAACTCTGCATGGATGTTTGCTTCTCGCCAGGGATTAG
CTGCTAGCGACATTAGGACATGGATGGGGGATTTCCGTAACATCAGAAATGTAG
CAAAATATGCTGCAAGACTTGGTCAGTCTTTTAGTTCCTCAACCGAAACATTAA
AGGTTCAGAAGTATGAGGTGGAAGAAATCTCAGATATAAAAAATGGTACACAGC
ATGTGTTCTCTGATGGGATTGGAAAGATATCATCTGCTTTTGCTAATGAAGTGGC
CATGAAGTGCAACCTGAAACGCTTTGCTCCTTCTGCTTTTCAAATAAGGTATGGT
GGTTATAAAGGTGTTGTTGCTGTTGACCCTACATCACGCTGGAAGCTTTCCTTAA
GAAAAAGCATGCTAAAGTTTCAGTCAGACAATATCACAGTAGATGTCCTTGCAT
ACAGCAAGTACCAGCCAGGCTTCCTTAATCGGCAGCTAATTACTCTTCTTTCGAC
ACTTGGAGTTAGAGATAGTGTCTTTGAACAGAAGCAAGAAGCAGTGAATC
AGTTGAACAAAATGGTTACTGATCCTCAAGCTGCTATCGAAGCAATTGAACTTAT
GCCCATGGGAGAAATAACAAATGCAGTTAAAGAGCTGTTGTTGTGTGGCTACCA
GCCTGATGATGAACCATATCTTTCTATGCTGCTACAAACTTTTAGGGCATCCAAA
TTACTAGAGCTGAAAACCAAGTCAAGGATATTAATCCCAAAAGGACGAGCAATG
ATGGGATGCTTGGATGAAACACGTACGCTGAAGTATGGGCAAGTGTTCATTCGA
GCTACTTCGGGTGTAAATGACAATGACAGGTTCACCGTGACAGGAAAAGTTGT
GATTGCCAAAAACCCTTGCCTCCACCCAGGTGATATACGGATTCTCCATGCTGTT
GATGTTCCTGTTTTGCACCACATGTTTAACTGTGTCGTCTTTCCACAGCAAGGAC

CAAGGCCGCATCCTAATGAGTGTTCAGGGAGTGATCTTGACGGGGACATATATTT
TGTTTCTTGGGACCCATCTCTCATTCCGCCTCGTATGGTGACACCTATGGACTATA
CTCCAGCACCAACAGAAACATTAGACCATGATGTTACAATTGAGGAAGTAGAAG
AGTACTTCACAAATTACATAGTCAATGAGAGTTTAGGAATGATTGCCAATGCACA
TGTAGTATTTGCGGATAAGGAAGACCTCAAGGCAGAGAGTTCACCATGCATTGA
GCTGGCCAAGCTCTTCTCCATTGCTGTTGACTTTCCCAAAACTGGAGTGCCAGC
CCTGATTCCTCCTGAACTACATGTGAAGGAGTATCCTGATTTCATGGAGAAGCTT
GACAAAGTCACCTATGAATCAAAAGGAGTAATCGGGAAGCTCTACAGGGAAAT
AAAGAAGCACACCCCTCACATAAAGCACTTCACAAGGGAGGTGGCAAGGCGG
TCTTATGACACCGATATGATTGTTGATGGCTATGAAGATTACATCACTGAAGCTAT
GGCATTGAAGGACGAGTACGACTTCAAATTGGGTAATCTTATGGATCACTATGG
AATAAAAAGTGAGGCCGAGATAATAAGTGGATGCATTCTTAAGATGGCAAAGAA
TTTCACAAAGAAAAGCGATGCTGATGCTATCAGATTGGCGGTGAGATCTTTGCG
TAAGGAAGCAAGGTCAAGGTTCAGTGAGATGAGCTTAGATGATAATGGGCATGG
CCATGATGCCTCCGAAGCCAAGGCATCCGCCTGGTACCATGTCACTTACCACCC
TGAATTCTGGGGTTGCTACAATGAAGGATATGAGCGACCACACTTTATCAGCTTT
CCATGGTGTATCTATGAGAAGCTTTTGCGCATTAAGCAAAGGAGGAAGTTCGTC
AGGAAGATGCAGCCCGAGTTGTTCTCTCTCCACAACTTGAGAATTTGA (SEQ ID
NO: 1)

The gene sequence of AtRDR1 is shown below:

ATGGGGAAGACAATTCAAGTGTTTGGATTCCCTAATGGTGTGAGCGCAGAAGA
AGTTAAGAAGTTTCTTGAAAGACTCACTGGTTCAGGCACTGTTTACGCAATCAA
AGTTAGACAACCAAAGAAAGGTGGTCCAAGAGTCTATGCCATTGTTCAATTCAC
ATCTGAGAGACATACGAGGCTGATCATCACTGCGGCAGCAGAACGTCTTTACTA
TGGAAGATCTTATCTCAAGGCTTTTGAAGTTGAACAAGACATTGTTCCAAAGCC
AAGAGCTTCATTACACACCATCTCTGGTTTAAAGATGTTCTTTGGATGTCAAGTC
TCGACTAAGAAGTTTTTAACTCTATGGAGTGCTCAAGATGTTTGTGTCTCGTTTG
GAATTGGGATGAGGAAACTGCATTTTTCGTTTTCTTGGTATCAAAAGGATTATCG
TCTTGAGCTTTCGTATGAGAATATATGGCAGATTGATTTGCATTCTCCGCAAGGA
CGATCATCGAAGTTTCTTGTGATTCAGGTGATTGGTGCTCCTAAAATATTTGAGA
AAGAGGATCAACCTATCAATCTTTTGTTTGGAATAATGGATTTTTACAGTGATGG
GTCTGATGAGCAATGGATTAGAACTACAGATTTCACTTCATCTTCTTGTATTGGT
CAATCAACAGCGTTTTGTTTAGAGCTTCCTGTTCATCTTAATGTTCCTGATTTCA

GAGAGAATTTTGCCAACTATGCAGAGCACAGAGCCAGCTCTTTTCTCATTGAAT
CTGGTTCTAGCTATTCATCTAATGCTAACACACTTGTTCCTGTTGTTGATCCTCCT
CCAGGGTTTAGTTTGCCTTTTGAGATCTTGTTTAAACTTAACACATTGGTGCAGA
ACGCGTGTCTCTCCGGGCCAGCTCTTGATCTTGACTTTTATCGGTTGCTTAATCA
GAAAAAATATGATAGAGCTCTCATAGATCACTGTCTTGAAAAACTCTTTCACCTT
GGAGAATGTTGCTATGAGCCTGCTCATTGGCTTCGCGATGAGTACAAGAAGTGG
ATATCGAAAGGAAAGCTTCCACTGTCTCCTACAATATCTCTTGATGATGGACTTG
TTTATATGTATCGCGTTCAAGTTACACCAGCAAGAGTCTATTTCTCTGGACCGGA
GGTGAATGTTTCTAATCGTGTGCTTCGACATTACTCCAAGTATATCAACAACTTC
CTCAGGGTCTCATTTGTTGATGAAGATCTTGAAAAGGTTCGTTCCATGGATCTAT
CTCCACGCTCTTCTACTCAAAGAAGAACCAAGTTATACGACAGGATATATTCTGT
TCTTCGCGATGGGATTGTCATAGGCGATAAGAAGTTTGAGTTTCTCGCCTTTTCT
TCCAGCCAGTTGCGGGAGAACTCTGCTTGGATGTTTGCACCAATAGATCGGATT
ACAGCAGCACATATCAGAGCTTGGATGGGTGATTTTGACCATATAAGAAATGTA
GCTAAATATGCTGCAAGGCTTGGTCAATCTTTTAGCTCTTCAAGAGAGACACTTA
ATGTTAGGAGTGATGAGATTGAAGTGATTCCTGATGTTGAGATTATATCTTTAGG
GACACGCTATGTGTTTTCCGATGGAATAGGAAAGATATCAGCTGAATTTGCTAGA
AAGGTAGCGAGGAAATGTGGTCTTACAGAGTTTTCTCCATCTGCTTTTCAAATC
CGTTATGGCGGTTATAAAGGAGTGGTGGCTGTTGATCCAAACTCATCAAAGAAA
CTGTCTCTGAGGAAGAGTATGAGCAAATTCGAATCGGAGAACACCAAGCTTGAT
GTTCTGGCGTGGAGCAAGTACCAACCTTGTTATATGAACAGACAACTGATTACA
CTTTTGTCTACTCTTGGAGTTACGGACAGTGTGTTTGAGAAGAAACAAAGGGA
AGTTGTGGATCGTCTCGACGCAATCTTGACTCATCCTTTGGAAGCTCACGAGGC
TCTTGGTTTAATGGCTCCAGGGGAAAACACAAATATTCTCAAGGCATTGATCTTG
TGTGGCTATAAACCTGACGCTGAACCTTTCCTTTCAATGATGCTTCAGAATTTCA
GGGCATCTAAGTTGTTGGAACTACGGACAAAAACAAGGATTTTCATTTCTGGTG
GAAGATCTATGATGGGATGCCTAGACGAGACCAGAACACTGGAGTATGGTCAGG
TAGTAGTGCAGTATTCAGATCCCATGAGGCCGGGAAGGCGATTCATCATCACCG
GACCTGTTGTTGTTGCCAAAAACCCATGCCTGCATCCTGGTGACGTGCGTGTTC
TTCAAGCTGTCAATGTCCCAGCTTTAAATCATATGGTGGACTGCGTTGTGTTCCC
GCAAAAGGGCTTGAGGCCACACCCAAATGAATGTTCTGGGAGTGATTTAGATG
GAGATATTTACTTTGTATGTTGGGATCAAGAATTGGTTCCGCCAAGAACGTCTGA
ACCAATGGACTACACTCCTGAACCAACTCAAATCTTGGATCATGATGTCACAATT

GAGGAAGTTGAAGAGTACTTTGCGAACTACATTGTGAATGATAGTTTAGGGATC
ATCGCGAATGCTCATACCGCCTTTGCGGATAAGGAACCACTCAAGGCGTTTAGT
GACCCATGCATTGAGCTTGCAAAGAAGTTTTCAACTGCGGTAGATTTCCCAAAA
ACTGGTGTTGCAGCTGTGATACCGCAACATCTTTACGTGAAAGAGTATCCTGATT
TCATGGAGAAACCGGATAAGCCAACATATGAGTCGAAGAACGTGATTGGTAAGC
TTTTTAGAGAGGTAAAAGAGCGAGCTCCACCGTTGATCTCGATCAAATCATTCA
CGCTTGATGTGGCCTCAAAGTCTTATGATAAAGACATGGAAGTTGACGGATTTG
AGGAGTATGTTGATGAAGCTTTCTACCAGAAGGCGAATTATGATTTCAAGTTAG
GTAATTTGATGGATTACTATGGGATTAAGACTGAGGCTGAGATTCTTAGTGGTGG
TATAATGAGGATGTCAAAGTCATTCACCAAGAGACGAGATGCAGAATCGATTGG
AAGAGCGGTTCGGGCGTTGAGGAAAGAAACTTTGTCGTTGTTCAATGCTTCTG
AAGAAGAAGAAAATGAGTCAGCAAAGGCTTCGGCTTGGTATCATGTAACGTAC
CACTCAAGTTACTGGGGACTTTATAATGAGGGTTTGAATCGTGACCATTTCTTAA
GTTTTGCGTGGTGCGTTTATGATAAGCTTGTGAGGATCAAGAAGACCAATCTTG
GGAGGCGTCAGCGGCAGGAGACGCTCGAGCGGTTAGACCATGTTCTGCGTTTC
GGTTGA (SEQ ID NO: 2)

The amino acid sequence of OsRDR1 is shown below:

MMDWFMSTVYFYGPEINVSNRVVRNFSSDIENFLRISFVDEDCEKLRATDLSPRSA
SGHDANRTALYKRVLSVLSDGITIGGKNFEFLAFSSSQLRDNSAWMFASRQGLAA
SDIRTWMGDFRNIRNVAKYAARLGQSFSSSTETLKVQKYEVEEISDIKNGTQHVFS
DGIGKISSAFANEVAMKCNLKRFAPSAFQIRYGGYKGVVAVDPTSRWKLSLRKSM
LKFQSDNITVDVLAYSKYQPGFLNRQLITLLSTLGVRDSVFEQKQEEAVNQLNKM
VTDPQAAIEAIELMPMGEITNAVKELLLCGYQPDDEPYLSMLLQTFRASKLLELKT
KSRILIPKGRAMMGCLDETRTLKYGQVFIRATSGVNDNDRFTVTGKVVIAKNPCL
HPGDIRILHAVDVPVLHHMFNCVVFPQQGPRPHPNECSGSDLDGDIYFVSWDPSLI
PPRMVTPMDYTPAPTETLDHDVTIEEVEEYFTNYIVNESLGMIANAHVVFADKEDL
KAESSPCIELAKLFSIAVDFPKTGVPALIPPELHVKEYPDFMEKLDKVTYESKGVIG
KLYREIKKHTPHIKHFTREVARRSYDTDMIVDGYEDYITEAMALKDEYDFKLGNL
MDHYGIKSEAEIISGCILKMAKNFTKKSDADAIRLAVRSLRKEARSRFSEMSLDDN
GHGHDASEAKASAWYHVTYHPEFWGCYNEGYERPHFISFPWCIYEKLLRIKQRRK
FVRKMQPELFSLHNLRI (SEQ ID NO: 3)

The amino acid sequence of AtRDR1 is shown below:

MGKTIQVFGFPNGVSAEEVKKFLERLTGSGTVYAIKVRQPKKGGPRVYAIVQFTSE
RHTRLIITAAAERLYYGRSYLKAFEVEQDIVPKPRASLHTISGLKMFFGCQVSTKKF
LTLWSAQDVCVSFGIGMRKLHFSFSWYQKDYRLELSYENIWQIDLHSPQGRSSKF
LVIQVIGAPKIFEKEDQPINLLFGIMDFYSDGSDEQWIRTTDFTSSSCIGQSTAFCLEL
PVHLNVPDFRENFANYAEHRASSFLIESGSSYSSNANTLVPVVDPPPGFSLPFEILFK
LNTLVQNACLSGPALDLDFYRLLNQKKYDRALIDHCLEKLFHLGECCYEPAHWLR
DEYKKWISKGKLPLSPTISLDDGLVYMYRVQVTPARVYFSGPEVNVSNRVLRHYS
KYINNFLRVSFVDEDLEKVRSMDLSPRSSTQRRTKLYDRIYSVLRDGIVIGDKKFEF
LAFSSSQLRENSAWMFAPIDRITAAHIRAWMGDFDHIRNVAKYAARLGQSFSSSRE
TLNVRSDEIEVIPDVEIISLGTRYVFSDGIGKISAEFARKVARKCGLTEFSPSAFQIRY
GGYKGVVAVDPNSSKKLSLRKSMSKFESENTKLDVLAWSKYQPCYMNRQLITLL
STLGVTDSVFEKKQREVVDRLDAILTHPLEAHEALGLMAPGENTNILKALILCGYK
PDAEPFLSMMLQNFRASKLLELRTKTRIFISGGRSMMGCLDETRTLEYGQVVVQY
SDPMRPGRRFIITGPVVVAKNPCLHPGDVRVLQAVNVPALNHMVDCVVFPQKGL
RPHPNECSGSDLDGDIYFVCWDQELVPPRTSEPMDYTPEPTQILDHDVTIEEVEEYF
ANYIVNDSLGIIANAHTAFADKEPLKAFSDPCIELAKKFSTAVDFPKTGVAAVIPQH
LYVKEYPDFMEKPDKPTYESKNVIGKLFREVKERAPPLISIKSFTLDVASKSYDKD
MEVDGFEEYVDEAFYQKANYDFKLGNLMDYYGIKTEAEILSGGIMRMSKSFTKR
RDAESIGRAVRALRKETLSLFNASEEEENESAKASAWYHVTYHSSYWGLYNEGLN
RDHFLSFAWCVYDKLVRIKKTNLGRRQRQETLERLDHVLRFG (SEQ ID NO: 4)

### Example 2. RDR1 protein can inhibit the proliferation of cancer cells

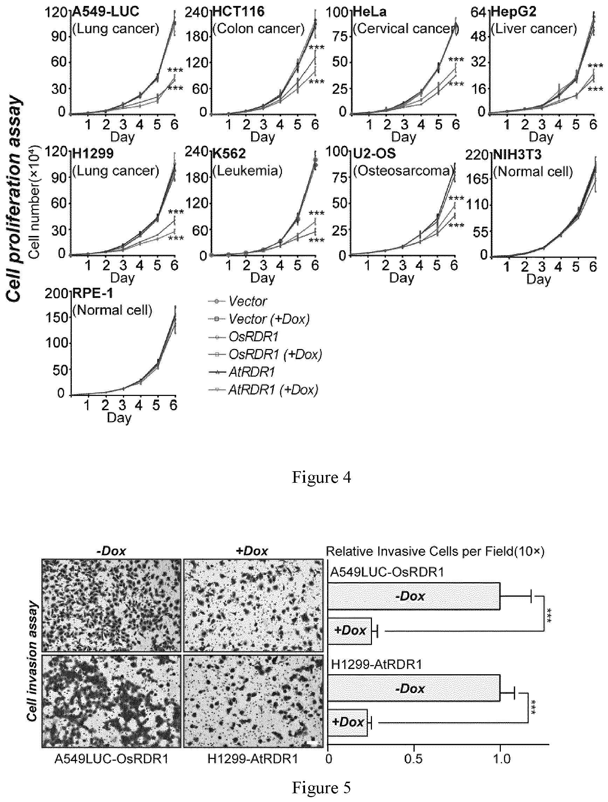

After the stable cell lines expressing the inducible RDR1 protein were obtained, the effect of the RDR1s on the proliferation of cancer cells was further investigated. Initially, 10,000 cells were plated in a 24-well plate or 12-well plate, and an experiment group was set as the high Dox group (4 µg/mL), and the cell lines without Dox or transfected with empty vector were set as the control group. The experiments were performed in triplicate for 6 consecutive days and the cells were counted according to trypan blue staining. The results showed that both rice RDR1 and *Arabidopsis thaliana* RDR1 could effectively inhibit the proliferation of various cancer cell lines with distinct tissue origins. For example, the growth of the cancer cells from cervix, lung, liver, etc. was significantly slowed, while the proliferation of normal cell line was substantially unaffected (Figure 4).

### Example 3. RDR1 protein can inhibit the invasion of cancer cells

Next, the effect of the RDR1s on the invasion of cancer cells was tested. In this example, the cell lines A549LUC-OsRDR1 and H1299-AtRDR1 constructed in Example 1 were selected, whereas A549LUC and H1299 are evidently invasive. Cell invasion was measured using a BioCoat Matrigel Invasion Chamber (Corning) following the manufacturer's instruction. After 5 days of induction with Dox, the invasion experiment was conducted. After 24 hours, cells were observed, photographed and counted. The results showed that either OsRDR1 or AtRDR1 remarkably reduced the number of cancer cells that invaded through the chamber, indicating RDR1 could significantly inhibit the invasion of cancer cells (Figure 5).

### Example 4. RDR1 protein does not affect the apoptosis of cancer cells

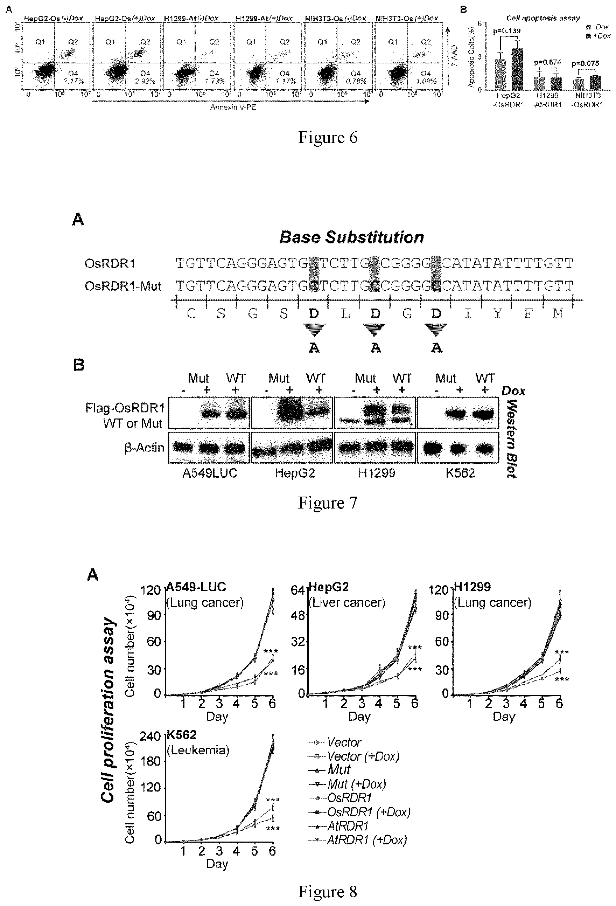

The apoptosis changes of cancer cell lines stablly transfected with OsRDR1 or AtRDR1 were further investigated. 10,000 cells were plated in a 6-well plate, high concentration of Dox (4 µg/mL) was added for the experiment group, and no Dox was added for the control group, three replicates each. Six days later, the experiments were carried out using Annexin V-PE/7-AAD Apoptosis Detection Kit (Vazyme) according to the instruction. The cells were digested with trypsin, washed by PBS, and then stained by 7-AAD and PE, respectively. The results were analyzed by FACS. As shown in Figure 6A and B, after induction of RDR1 expression by Dox for 6 days, no obvious apoptosis was seen for tumor cells, suggesting the proliferation slowdown caused by RDR1 overexpression was not due to induction of apoptosis.

### Example 5. RDR1 mutations lead to the loss of cancer suppressive function

In order to further verify the function of the RDR1 protein, the loss-of-function RDR1 mutants were prepared in this example. First, RDR1 gene sequences in plants, microorganisms and other species were searched on NCBI and Uniprot and downloaded, before subject to alignment using DNAMAN. The identified conserved domains are shown in Figure 1. Subsequently, by using Q5 Site-Directed Mutagenesis Kit (NEB), PCR primers Mut_RDR1_F (SEQ ID NO: 20) and Mut_RDR1_R (SEQ ID NO: 21) were designed following the instruction, and confirmed by sequencing. The virus OsRDR1-Mut was packaged according to the method described in 4.1 (Figure 7A). After packaging, distinct cancer cell lines were infected, and the positive cells were flow-sorted after induction with low concentration of Dox (100 ng/mL) for 24 hours. Expression of OsRDR1-Mut was detected by Western Blot after expansion, and then compared with wild-type RDR1 expression to exclude the possibility that loos-of-function mutants were resulted from mutant protein degradation (Figure 7B).

Finally, cell proliferation experiment was performed for the OsRDR1-Mut cell line according to the method described in Example 2. The results showed that after RDR1 protein was mutated, the mutant RDR1 could not inhibit the proliferation of cancer cells, even if the over-expression of RDR1-Mut in cancer cells was induced by high concentration of Dox (Figure 8). The results of this example has demonstrated that RDR1's impact on cancer cells verified in previous examples is attributed to RDR1 function rather than other non-specific factors.

The amino acid sequence of OsRDR1-Mut is shown below:

MMDWFMSTVYFYGPEINVSNRVVRNFSSDIENFLRISFVDEDCEKLRATDLSPRSA
SGHDANRTALYKRVLSVLSDGITIGGKNFEFLAFSSSQLRDNSAWMFASRQGLAA
SDIRTWMGDFRNIRNVAKYAARLGQSFSSSTETLKVQKYEVEEISDIKNGTQHVFS
DGIGKISSAFANEVAMKCNLKRFAPSAFQIRYGGYKGVVAVDPTSRWKLSLRKSM
LKFQSDNITVDVLAYSKYQPGFLNRQLITLLSTLGVRDSVFEQKQEEAVNQLNKM
VTDPQAAIEAIELMPMGEITNAVKELLLCGYQPDDEPYLSMLLQTFRASKLLELKT
KSRILIPKGRAMMGCLDETRTLKYGQVFIRATSGVNDNDRFTVTGKVVIAKNPCL
HPGDIRILHAVDVPVLHHMFNCVVFPQQGPRPHPNECSGSALAGAIYFVSWDPSLI
PPRMVTPMDYTPAPTETLDHDVTIEEVEEYFTNYIVNESLGMIANAHVVFADKEDL
KAESSPCIELAKLFSIAVDFPKTGVPALIPPELHVKEYPDFMEKLDKVTYESKGVIG
KLYREIKKHTPHIKHFTREVARRSYDTDMIVDGYEDYITEAMALKDEYDFKLGNL
MDHYGIKSEAEIISGCILKMAKNFTKKSDADAIRLAVRSLRKEARSRFSEMSLDDN
GHGHDASEAKASAWYHVTYHPEFWGCYNEGYERPHFISFPWCIYEKLLRIKQRRK
FVRKMQPELFSLHNLRI (SEQ ID NO: 5)

### Example 6. Truncated RDR1 effectively inhibits the proliferation of cancer cells

Based on the mutations described in Example 5, OsRDR1 and AtRDR1 were truncated in this example. On one hand, it was further investigated whether the inhibitory effect of RDR protein on cancer was mediated by its key enzyme domain; on the other hand, given the conservation of RDRp domain, inhibiton of cell proliferation with this single key domain of RDR was investigated.

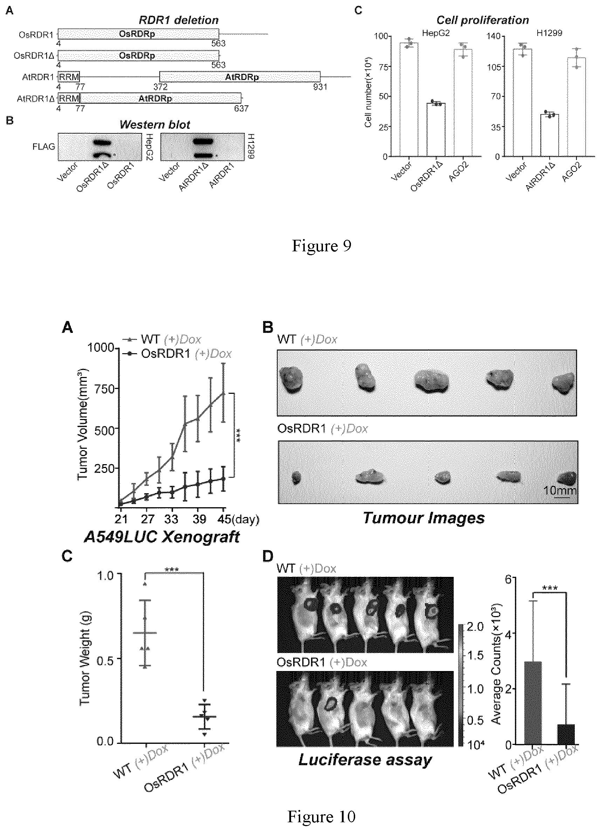

Domain information of OsRDR1 and AtRDR1 was first obtained by searching in SMART database. Subsequently, by using Q5 Site-Directed Mutagenesis Kit (NEB), PCR primers OsRDRp F (SEQ ID NO: 26) and OsRDRp R (SEQ ID NO: 27) were designed according to the instruction to amplify the truncated RDR1 sequence, which was comfirmed by sequencing, as shown schematically in Figure 9A. In this example, OsRDR1 truncation was obtained by removal of C-terminus containing no definite functional domains, and AtRDR1 truncation was obtained by removal the region between RRM (RNA recognition motif) and AtRDRp as well as the C-terminus containing no definite functional domains. Subsequently, we transferred the vectors containing FLAG-OsRDR1Δ and AtRDR1Δ into liver cancer cells HepG2 and non-small cell lung cancer cells H1299, respectively, using lipo3000-mediated transient transfection. After 84 hours, cells were collected and detected by counting to determine cell proliferation. Expression of OsRDR1Δ and AtRDR1A was detected by Western Blot, and compared with that of control cells transiently transfected with full-length wild-type RDR1 proteins.

The results showed that after liposome-mediated transient transfection, OsRDR1Δ and AtRDR1Δ, two truncated RDR1s consisting of single RDRp domains, were successfully expressed in cancer cells; however, full-length protein was not detected (Figure 9B) in the cells transiently transfected with full-length protein. The cause might be this protein is too long or there exist certain sites flanking RDRp domain which could promote protein degradation (Figure 9B).
The amino acid sequence of OsRDR1Δ is as follows: 
MMDWFMSTVYFYGPEINVSNRVVRNFSSDIENFLRISFVDEDCEKLRATDLSPRSA
SGHDANRTALYKRVLSVLSDGITIGGKNFEFLAFSSSQLRDNSAWMFASRQGLAAS
DIRTWMGDFRNIRNVAKYAARLGQSFSSSTETLKVQKYEVEEISDIKNGTQHVFSD
GIGKISSAFANEVAMKCNLKRFAPSAFQIRYGGYKGVVAVDPTSRWKLSLRKSMLK
FQSDNITVDVLAYSKYQPGFLNRQLITLLSTLGVRDSVFEQKQEEAVNQLNKMVTD
PQAAIEAIELMPMGEITNAVKELLLCGYQPDDEPYLSMLLQTFRASKLLELKTKSRI
LIPKGRAMMGCLDETRTLKYGQVFIRATSGVNDNDRFTVTGKVVIAKNPCLHPGDI
RILHAVDVPVLHHMFNCVVFPQQGPRPHPNECSGSDLDGDIYFVSWDPSLIPPRMV
TPMDYTPAPTETLDHDVTIEEVEEYFTNYIVNESLGMIANAHVVFADKEDLKAESS
PCIELAKLFSIAVDFPKTGVPALIPPELHVKEYPDFMEKLDKVTYESKGVIGKLYREI
 (SEQ ID NO: 6)
The amino acid sequence of AtRDR1Δ is as follows:

MGKIQVFGFPNGVSAEEVKKFLERLTGSGTVYAIKVRQPKKGGPRVYAIVQFTSER
HTRLIITAAAERLYYGRSYLVTPARVYFSGPEVNVSNRVLRHYSKYINNFLRVSFV
DEDLEKVRSMDLSPRSSTQRRTKLYDRIYSVLRDGIVIGDKKFEFLAFSSSQLRENS
AWMFAPIDRITAAHIRAWMGDFDHIRNVAKYAARLGQSFSSSRETLNVRSDEIEVI
PDVEIISLGTRYVFSDGIGKISAEFARKVARKCGLTEFSPSAFQIRYGGYKGVVAVD
PNSSKKLSLRKSMSKFESENTKLDVLAWSKYQPCYMNRQLITLLSTLGVTDSVFE
KKQREVVDRLDAILTHPLEAHEALGLMAPGENTNILKALILCGYKPDAEPFLSMM
LQNFRASKLLELRTKTRIFISGGRSMMGCLDETRTLEYGQVVVQYSDPMRPGRRFI
ITGPVVVAKNPCLHPGDVRVLQAVNVPALNHMVDCVVFPQKGLRPHPNECSGSD
LDGDIYFVCWDQELVPPRTSEPMDYTPEPTQILDHDVTIEEVEEYFANYIVNDSLGI
IANAHTAFADKEPLKAFSDPCIELAKKFSTAVDFPKTGVAAVIPQHLYVKEYPDFM
EKPDKPTYESKNVIGKLFREV (SEQ ID NO: 7)

Furthermore, cell counting showed that cell proliferation was notebly reduced ~3.5 days after transfected with OsRDR1Δ and AtRDR1Δ, respectively, compared with control cells transfected with empty vector and AGO2-carrying vector, respectively, suggesting the cancer-inhibiting function of RDR1 protein was mediated by its RDRp domain, and RDRp domain alone was enough to inhibit tumor cell proliferation (Figure 9C).

The significance of this example is that: in light of aforementioned content, RDRp is extremely conservative across all RDR genes of distinct species (not limited to RDR1). Given RDRp alone can achieve the purpose of the present disclosure, genes belonging to RDR familes from each and every species is anticipated to produce similar technical effects. Meanwhile, by replacing full-length protein with RDRp domain, a shorter fragment, the protein stability is improved, thereby improving the developability for future drug development, making it extremely valuable for clinical use.

### Example 7. RDR1 protein can inhibit tumor growth in tumor-bearing mice model

In order to further study the inhibitory effect of RDR1 on cancer cells, the cell line A549LUC was selected for the tumor-bearing model experiment in this example. Wild-type A549LUC cells and A549LUC-OsRDR1 cells transfected with rice RDR1 were inoculated to NPG mice at equal amount (n=5), both of which were given Dox-containing drinking water, and tumor volume was consecutively measured using a calliper. When the tumor in the control group reached 700-800 mm³ in volume, it was taken and weighed. The results was shown in Figure 10A, B and C. Over-expression of the RDR1 could significantly reduce the volume and weight of the tumors formed with A549LUC-OsRDR1 cells. Meanwhile, the possibility of tumor inhibiton by Dox was also ruled out, since both groups were given Dox in drinking water at the same time. Figure 10D showed that RDR1 reduced the luciferase intensity of A549 tumor. Moreover, As shown in this example, the inhibitory effect of the RDR1 was persistently potent after 21 days when tumor was well formed. Tumor growth was continously slowing down from the perspective of growth trend, and tumor even stopped growing when experiment ended. These results indiate RDR1 could significantly and persistently inhibit tumor growth *in vivo.*

### Example 8. RNA expression analysis in the cancer cells expressing the RDR1 protein

In order to investigate the mechanism of RDR1 inhibiting cancer cell proliferation, in this example, different cell lines continuously induced to express RDR1 for 6 days were further studied. After RNA extraction using Trizol, library was constructed according to the instruction of Illumina RNA-seq kit, before subjected to NGS sequencing. Sequencing data were processed and aligned to obtain the expression level of each gene in the cell lines.

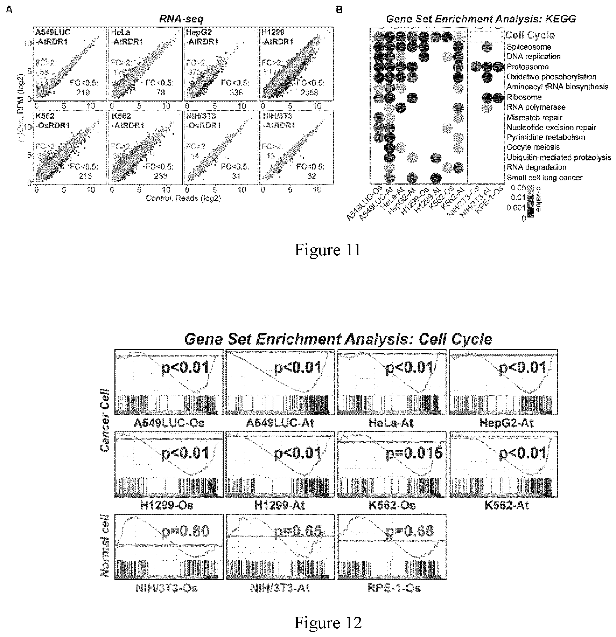

Subsequently, gene expression change resulted from the induction of stable RDR1 expression was observed at the transcriptome level in different cancer cell lines. With normal cell line serving as control, 2-fold change in gene expression was determined as the threshold of significance. For the same cell line, wild-type or uninduced group served as reference. The results showed that RDR1 significantly altered the overall transcriptome expression level in cancer cells (Figure 11A), while caused minor change in normal cell lines, i.e., NIH/3T3 and RPE-1, which was consistent with previous observation that RDR1 specifically curbed cancer cells, rather than normal cells. Meanwhile, more down-regulated genes than up-regulated genes could be observed in varieties of cancer cells, which was also consistent with the inhibitory effect of RDR1 on cancer cells as previously described.

In order to further investigate the pathways involved in producing the inhibitory effect of RDR1 on cancer cells proliferation, cluster analysis was further performed in this example using GSEA genome-wide data. The gene set of KEGG was selected as the reference for GSEA analysis. The gene expression list and data set were imported into the software according to the instruction, and the significantly changed pathways were screened using FDR<0.25, p<0.05, as recommended in the official GSEA document. Figure 11B shows the significantly down-regulated pathways in various cancer cells expressing RDR1. Cell cycle related pathways were significantly down-regulated across all cancer cell lines, but not in normal cell lines. Meanwhile, the DNA replication related pathways were observed to be remarkably down-regulated in most cancer cell lines as well. Notably, these two pathways were usually considered to be relevant to tumor cell proliferation.

Furthermore, compiled analysis was conducted separately for the cell cycle pathway results picked out from the source file of the kegg analysis in Example 7. Figure 12 showed both OsRDR1 and AtRDR1 significantly reduced the overall expression of cell cycle-related genes (black lines were generally clustered toward the right side) in a variety of cancer cell lines, including non-small cell lung cancer cell line A549LUC and H1299, cervical cancer cell line HeLa, liver cancer cell line HepG2, and chronic myeloid leukemia cell line K562. While in normal cell lines, no apparant tendency for the regulation of gene expression was observed (black lines were generally clustered toward the left side). This demonstrates that RDR1 does not influence the cell cycle in normal cells, but specifically targets the abnormally activated genes in cancer cells.

### Example 9. RDR1 alters the cell cycle in cancer cells

Using the aforementioned induction method for the 6-well plate, after 6 days of Dox induction, cells were digested with trypsin, fixed with 4% paraformaldehyde, treated with 0.3% Triton-X to disrupt cell membrane, stained according to the instruction of BeyoClick EdU-488 (Beyotime) kit, and immediately subjected to cell cycle analysis with flow cytometry. The results showed that after RDR1 protein was over-expressed for 6 days, compared with wild-type cells (uninfected) and no-Dox-induced cell line (no RDR1 over-expression), in cancer cells, including non-small cell lung cancer cell H1299, liver cancer cell HepG2 and colorectal cancer cell HCT116, most cells were arrested in G1 or G2/M phase of cell cycle, the proportion of cells in S phase reduced sharply, the proportion of cells in G1 phase increased substantially, and cells were also blocked in G2 phase to a certain extent. However, RDR1 had no significant effect on normal cell NIH/3T3. The above indicates that one of the mechanisms underlying how RDR1 protein specifically and broadly suppresses cancer is primarily the inhibition of bnormally expressed cell cycle related genes in the cancer cells, thereby blocking these cancer cells in G1 and G2/M phases so as to reduce the proportion of the cells in S phase.

### Example 10. RDR1 changes the expression of proto-oncogene in cancer cells and related experimental verification thereof

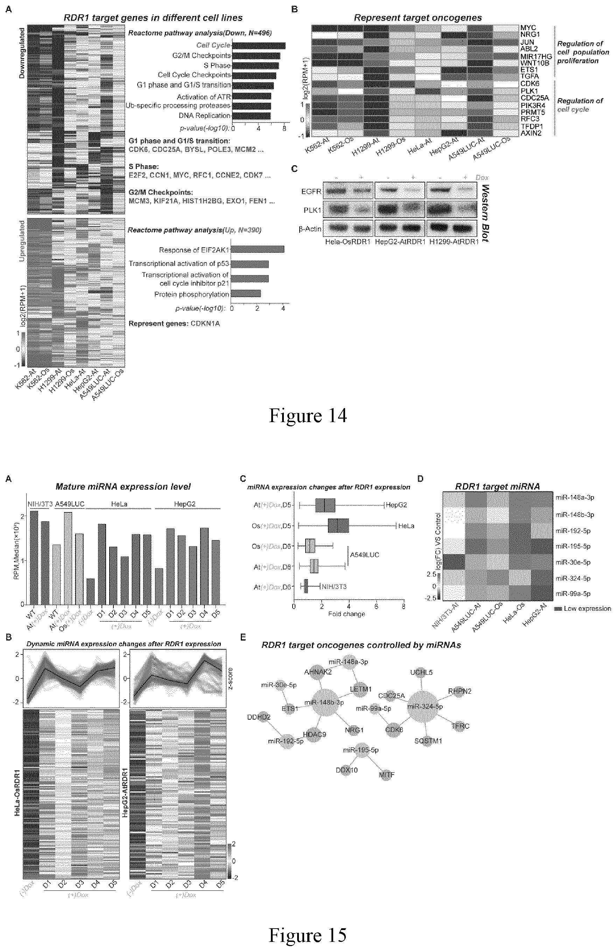

The results of the foregoing examples demonstrate that introduction and induced expression of RDR1 protein in multiple cancer cell lines lead to a significant down-regulation of overall expression of some carcinogenesis related pathways, suggesting some down-regulated genes may be shared among these pathways, which might have profound impact on the phenotype. For such genes, the k-means clustering method was used to identify the commonly down-regulated genes from the heatmap of gene expression profile for a plurality of cell lines, and clustering was achieved using the built-in clustering analysis tool in Reactome. Figure 14A showed the clustering results of the co-downregulated genes. Reactome is an open-source database that includes signaling pathways and metabolic molecules as well as the biological pathways and processes in which they are involved. As shown by the analysis results, among the pathways where 496 down-regulated genes were significantly enriched, 8 pathways with the lowest p-value mainly comprised cell cycle pathways and their sub-pathways, including known proto-oncogenes or crucial genes for the regulation of cell proliferation, such as CKD6, MYC, etc.. As shown in the present example, genes targeted and down-regulated by RDR1 in different cancer cells belonged to cell cycle pathway, which was consistent with the results of GSEA analysis, further confirming that RDR1 inhibited the abnormal proliferation of cancer cells by regulating the expression of the cell cycle genes.

Further, through literature search and database retrieval, 49 proto-oncogenes were identified from the 496 commonly down-regulated genes. The proto-oncogenes among the target genes were annotated by GO analysis, and it was found that these genes were mainly divided into two categories, i.e., the proto-oncogenes regulating cell cycle and the proto-oncogenes regulating the proliferation of cell population (Figure 14B).

From the heatmap of the oncogenes that were commonly significantly down-regulated, oncogenes having relatively significant effect and intensely investigated were selected for Western Blot. 10,000 cells were plated in a 6-well plate. For the experiment group, Dox was added at high concentration (4 µg/mL), while for the control group, no Dox was added. Cells were continuously induced to over-express RDR1 for 6 days, harvested, treated with RIPA lysis solution and PMSF, centrifuged to obtain supernatant and detected by Western Blot for the expression of selected marker genes in the cancer cells (Figure 14C). After RDR1 was over-expressed by induction with Dox for 6 days, both epidermal growth factor EGFR and the oncogene PLK1 which regulates cell cycle were found to be down-regulated to a certain extent at the protein level, which was consistent with aforementioned RNA-seq data, further confirming that the overexpression of RDR1 protein could inhibit the abnormally expressed oncogenes and cell cycle-related genes in cancer cells.

### Example 11. RDR1 can increase the miRNAs down-regulated in cancer cells

In plant cells, the innate immunity of plant is realized by the production of small RNAs mediated by plant immune proteins with RDR1 as the core. In order to further investigate the possible influence of RDR1 on the small RNA pathways in animal cells, in this example, small RNA-seq was performed with cancer cells expressing RDR1 protein to find out whether RDR1 protein affected small RNA pathways in cancer cells, thereby inhibiting the proliferation and invasion of cancer cells.

First, non-small cell lung cancer cells A549LUC-OsRDR1/AtRDR1 and normal cells NIH3T3-AtRDR1 were collected after induced expression for 6 continuous days. The cervical cancer cells HeLa-OsRDR1 and the liver cancer cells HepG2-AtRDR1 were collected at a fixed interval of 24 h (the induction culture medium containing Dox was added on the first day, and the cells were collected at a fixed interval of 24 h; cells in the medium containing no Dox were used as the control). RNA was extracted from these cells. 30 µg of total RNA was obtained after separation on a 15% TBE-urea gel (Invitrogen), and the small RNAs with a length of 15-50 nt were recovered from the gel. Subsequently, a library was constructed using NEBNext^{®} Small RNA Library Prep Set (NEB). The constructed library was then subjected to NGS sequencing. Sequencing data were furter analyzed after removal of adapter and sequence alignment.

Analysis of the small RNA-seq data of the cells from each group showed that after the expression of RDR1 in various cancer cell lines, the overall expression of miRNAs in cancer cells were up-regulated, which is even more pronounced in HeLa-OsRDR1 and HepG2-AtRDR1 (during the 5-day interval, miRNA expression increased steadily and fluctuantly, compared with control). However, there was no significant change in normal cells NIH/3T3 (Figure 15A-C). This indicates RDR1 could restore the expression of these unbalanced miRNAs in cancer cells. While miRNA production in normal cells was strictly regulated, thus RDR1 will not cause "accidental injury" to the normal cells.

Mature miRNAs that were significantly up-regulated compared to corresponding control groups (at least 2 folds of the control group) in three data sets for A549-AtRDR1, A549-OsRDR1, HeLa-OsRDR1 and HepG2-AtRDR1 were picked out to form a dataset, and target gene prediction was carried out using miRWalk. The intersection was acquired between the proto-oncogenes obtained in Example 10 and the genes predicted by miRWalk. Figure 15D showed the miRNAs from RDR1 target genes. These miRNAs was remarkably up-regulated due to RDR1, thereby enhancing the regulation of their target genes.

Through analysis of these target miRNAs, it could be seen that miRNAs that were commonly significantly up-regulated in various cancer cells correlated and corresponded well to the oncogenes that were targeted by RDR1 (Figure 15D-E). Thus, the expression of RDR1 in these cancer cells might target the over-activated proto-oncogenes by increasing the expression of these miRNAs. Therefore, RDR1 inhibits the proliferation of the cancer cells through the "RDR1-miRNA-proto oncogenes-cell cycle" axis.

### Example 12. RDR1 added tail to miRNAs in cancer cells by increase the expression of down-regulated miRNAs

Expression of RDR1 in cancer cells appeared to restore the expression of down-regulated miRNAs in the cancer cells, as revealed by small RNA sequencing. However, how RDR1 up-regulates the miRNAs of the target genes is still unclear. Meanwhile, as an RNA-dependent RNA polymerase, the biochemical mechanism of the RDR1 still needs to be further elucidated. A study on RDR6 protein showed that RDR6 had 3' nucleotidyl transferase activity and the RNA synthesis activity using single-stranded RNA as template. However, synthesis of miRNA with RDR6 using miRNA as template has not been reported. A possible mechanism is that the 3' nucleotidyl transferase of RDR1 adds tail to miRNA, which may promote miRNA processing and maturation .

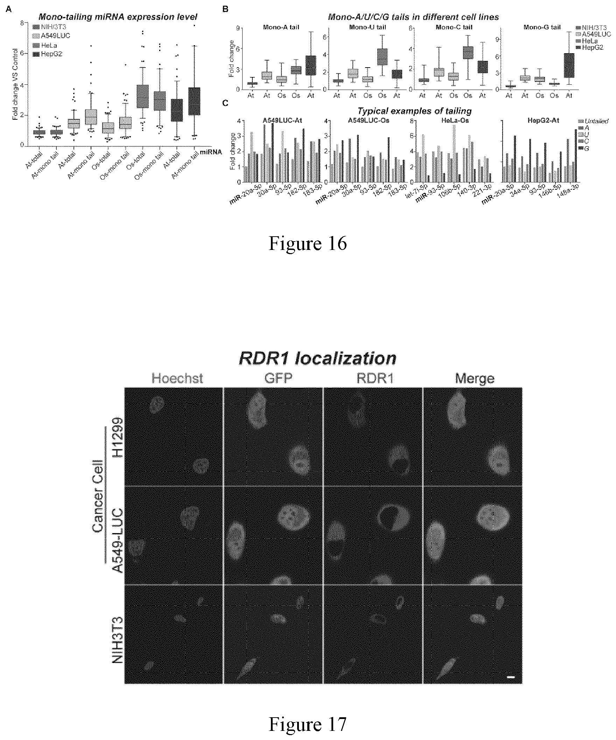

In order to investigate miRNA tailing, with reference to small RNA-seq processing method, statistic analysis was performed in this example on the cell line A549LUC and HepG2, which exhibited apparent tailing. Figure 16A showed that the proportion of tailing rises with time in each cancer cell line. Considering the results of Example 11, RDR1 increased the overall expression of miRNAs that were down-regulated in cancer cells. In fact, the increase in the amount of tailed miRNAs was greater than the increase in miRNA expression, so that the tailed miRNAs in Figure 16A accounted for a higher proportion.

In order to find out whether the tailing was nucleotide-biased, statistic analysis was performed for the tailing of four single nucleotides, and compared with those without tailing. Figure 16B showed the statistical results of these four nucleotide tailings in A549, HepG2 and NIH/3T3.

The results showed that in HepG2-AtRDR1, tailing with four nucleotides were all elevated, and there was a clear preference for A-tailing and G-tailing. Tailed miRNAs has obviously higher rise than un-tailed miRNAs. In A549LUC-OsRDR1, the increase in the expression of mature miRNAs having single nucleotide tail as well as miRNAs without tail was slightly less than that in A549LUC-AtRDR1. The increase of A-tailed and G-tailed miRNAs was slightly greater than the other two types of tailing. While in HeLa-OsRDR1, U-tailed and C-tailed miRNAs were preferred. Notably, for all types of tailing, there was no significant change in NIH/3T3 with the amount of single nucleotide tailed miRNAs and miRNAs without tail (Figure 16B). Figure 16C showed the tailing of some clearly identified miRNAs that were expressed in relative great amount, which once again confirmed the analysis in Figures 15 and 16B that RDR1 tailed miRNAs in cancer cells to increase the expression of these originally down-regulated miRNAs.

### Example 13. RDR1 taiiled miRNAs in cancer cells

In order to verify the conclusion of the small RNA-seq analysis, a cell line comprising OsRDR1-mcherry fusion protein was constructed to determine the localization of RDR1 protein inside cells. The sequence of mcherry was fused before the C-terminal stop codon of OsRDR1 using primers location_OsRDR1_mcherry_F (SEQ ID NO:22), location _OsRDR1_mcherry_R (SEQ ID NO:23), location_T2A-EGFP_F (SEQ ID NO:24) and location_T2A-EGFP_R (SEQ ID NO:25), ligated to the lentiviral vector together with with the T2A-EGFP sequence. After sequencing confirmation, virus comprising OsRDR1-mcherry was packaged and infected cancer cells together with TetOn-3G virus. Cells were treated with Dox at low concentration (100 ng/mL) for induction. After cultured for 24h, cells were sorted by flow cytometry for EGFP and mcherry dual-positive cells, which was then expanded. 10,000 cells were plated on a specialized imaging dish, induced by adding a high concentration of Dox (4 µg/mL) for 48h, treated with 1:100 diluted Hoechst dye solution to stain the nuclei for half an hour, washed with PBS, and recorded by photos. The results showed that RDR1 was specifically localized in the cytoplasm rather than the nucleus, in both normal cells and cancer cells (Figure 17). This suggested that RDR1 protein elevated miRNA levels by miRNA A-tailing or G-tailing in the cytoplasm.

Next, it was further explored in this example whether the up-regulation of the miRNA levels by RDR1 protein in cancer cells via miRNA tailing was directly achieved by its own nucleotransferase activity, or indirectly achieved by interacting with other known proteins. In general, after Flag-IP (Immunoprecipitation) using the Flag tag attached to RDR1, the eluted supernatant containing the RDR1 protein and possibly interacting proteins was subjected to MS (Mass Spectrometry), to identify possible interacting proteins. Specifically, two plates of Hela and HepG2-OsRDR1/AtRDR1 cells with Dox (high concentration)-induced RDR1 over-expression were harvested from 15 cm culture dishes, washed with PBS before supplemented with 1 mL of prepared cell lysis solution and protease inhibitors, scraped and lysed on ice for 30 minutes, collected by centrifugation, supplemented with washed and treated ANTI-FLAG M2 agarose beads (Sigma-Aldrich) to conjugate overnight at 4 °C. The prepared 1×Flag peptide (Sigma-Aldrich) was added on the next day for competitive elution. After elution, the eluate containing 15 µg total protein was sent to the company for MS analysis. The results showed that the proteins that interacted with RDR1 identified in the above experiments were all internal reference proteins, and no protein related to miRNA production and processing pathway was found (Table 1). As shown in this example, RDR1 protein added one or more nucleotides to the 3'-terminus of the miRNAs through its own nucleotransferase activity.

Herein, through MS analysis after RDR1-IP and intracellular localization of RDR1, it has been confirmed that RDR1 protein broadly up-regulates miRNA levels by A- or G-tailing, which is a biological process independently occurred in cytoplasm, and does not depend on any other elements for miRNA production or modification pathways (Figure 17, Table 1). This finding is consistent with the conclusion of the small RNA analysis.

The above results have established a model for RDR1 to inhibit the proliferation of cancer cells and to treat the cell proliferative diseases: after introduction into cancer cells, miRNA tailing is broadly occurred by RDR1 through its 3' nucleotidyl transferase activity. These tailings are good for the stabilization and processing of miRNAs, which result in broadly increased miRNAs in cancers. However, due to the feedback mechanism of miRNA production in normal cells, RDR1 can not significantly alter the miRNA levels in the normal cell. In cancer cells, the production of miRNAs is generally inhibited due to various causes, and some genes (especially those related to cell proliferation) are over-activated in the cancer cell line, manifesting vigorous metabolic and proliferation activities. Increased miRNA expression by RDR1 will target and inhibit the genes that are significantly up-regulated in cancer cells, including many genes related to the cell cycle and the proliferation of cell population, resulting in specific and broad inhibition of cancers.

**Table 1. MS analysis of possible interacting proteins with RDR1 in cells**

| Hela-OsRDR1 (+)Dox | | | |
|---|---|---|---|
| Name of protein | Accession No. | Length of peptide | Coverage (%) |
| OsRDR1 | Q0DXS3 | 22 | 41 |
| TUBB | P07437 | 15 | 42 |
| TUBB4B | P68371 | 14 | 38 |
| TUBA1B | P68363 | 12 | 40 |
| TUBAIC | Q9BQE3 | 11 | 35 |
| HSPA8 | P11142-1 | 17 | 36 |
| FLNA | P21333 | 20 | 12 |
| SPTAN 1 | Q13813-2 | 16 | 9 |
| PRMT5 | 014744 | 15 | 37 |
| EIF4B | P23588 | 9 | 26 |
| SPTBN1 | Q01082-1 | 16 | 10 |
| KRT1 | P04264 | 14 | 32 |
| HSPA1A | P0DMV8 | 13 | 26 |
| ACTB | P60709 | 10 | 40 |

| Hela-AtRDR1 (+)Dox | | | |
|---|---|---|---|
| Name of protein | Accession No. | Length of peptide | Coverage (%) |
| FLNA | P21333 | 36 | 20 |
| EIF4B | P23588 | 20 | 33 |
| AtRDR 1 | A0A178WKG4 | 21 | 25 |
| SPTAN 1 | Q13813 | 29 | 16 |
| HSPA8 | P11142-1 | 15 | 35 |
| ACTG1 | P63261 | 13 | 50 |
| WDR77 | Q9BQA1 | 10 | 44 |
| SPTBN1 | Q01082-1 | 22 | 12 |
| TUBA1A | Q71U36 | 11 | 36 |
| PRMT5 | 014744 | 12 | 29 |
| TUBB | P07437 | 10 | 29 |
| TUBAIC | Q9BQE3 | 10 | 31 |
| TUBB4B | P68371 | 9 | 25 |
| HSPA1A | P0DMV8 | 10 | 17 |

| HepG2-OsRDR1 (+)Dox | | | |
|---|---|---|---|
| Name of protein | Accession No. | Length of peptide | Coverage (%) |
| ACTG1 | P63261 | 25 | 76 |
| ACTB | P60709 | 25 | 76 |
| SPTBN1 | Q01082-1 | 81 | 48 |
| SPTAN 1 | Q13813-3 | 85 | 47 |
| OsRDR 1 | Q0DXS3 | 35 | 51 |
| PRMT5 | 014744 | 31 | 61 |
| POTEE | Q6S8J3 | 9 | 11 |
| TUBB | P07437 | 24 | 66 |
| HSPA8 | P11142-1 | 34 | 68 |
| WDR77 | Q9BQA1 | 15 | 64 |
| TUBA1B | P68363 | 19 | 62 |
| TUBB4B | P68371 | 25 | 66 |
| FLNA | P21333 | 50 | 32 |
| TUBA1C | Q9BQE3 | 17 | 55 |

| HepG2-AtRDR1 (+)Dox | | | |
|---|---|---|---|
| Name of protein | Accession No. | Length of peptide | Coverage (%) |
| ACTG1 | P63261 | 22 | 73 |
| SPTAN 1 | Q13813-2 | 83 | 44 |
| SPTBN1 | Q01082-1 | 72 | 45 |
| PRMT5 | 014744 | 31 | 59 |
| WDR77 | Q9BQA1 | 15 | 66 |
| POTEE | Q6S8J3 | 7 | 9 |
| AtRDR 1 | A0A178WKG4 | 34 | 40 |
| ACTC1 | P68032 | 11 | 35 |
| VIL1 | P09327 | 26 | 46 |
| FLNA | P21333 | 38 | 27 |
| HSPA8 | P11142-1 | 27 | 56 |
| EIF4B | P23588 | 17 | 37 |
| ACT7 | P53492 | 4 | 15 |
| DBN1 | 016643-3 | 15 | 36 |

**Table 2. All primer sequences used in the examples**

| Primer name | Sequence |
|---|---|
| AtRDR1_Sall_F | GGTCGACATGGGGAAGACAATTCAAGTGTT (SEQ ID NO: 8) |
| AtRDR1_BamH1_R | CGGATCCTCAACCGAAACGCAGAACATGGTC (SEQ ID NO: 9) |
| OsRDR1_Sall_F | GGTCGACATGATGGATTGGTTTATGTCTAC(SEQ ID NO: 10) |
| OsRDR1_BamH1_R | CGGATCCTCAAATTCTCAAGTTGTGGAG (SEQ ID NO: 11) |
| plenti_AtRDRl_CDS_F | TGCGGTACCGCGGGCATGGGGAAGACAATTCAAGTGTTTG(SEQ ID NO:12) |
| plenti_AtRDRl_CDS_R | ACTTCCTCTGCCCTCACCGAAACGCAGAACATGGTCT(SEQ ID NO: 13) |
| plenti_AtRDR1_T2A-EGFP_F | GTTCTGCGTTTCGGTGAGGGCAGAGGAAGTCTTCTAA(SEQ ID NO: 14) |
| plenti_AtRDR1_T2A-EGFP_R | CGACTCTAGAGTCGCTTACTTGTACAGCTCGTCCATG(SEQ ID NO: 15) |
| plenti_OsRDRl_CDS_F | TGCGGTACCGCGGGCATGATGGATTGGTTTATGTCTACAG(SEQ ID NO: |
| plenti_OsRDRl_CDS_R | 16) ACTTCCTCTGCCCTCAATTCTCAAGTTGTGGAGAGAGAACA(SEQ ID NO: 17) |
| plenti_OsRDR1_T2A-EGFP_F | CACAACTTGAGAATTGAGGGCAGAGGAAGTCTTCTAA(SEQ ID NO: 18) |
| plenti_OsRDR1_T2A-EGFP_R | CGACTCTAGAGTCGCTTACTTGTACAGCTCGTCCATG(SEQ ID NO: 19) |
| Mut_RDR1_F | GAGGGCAGAGGAAGTCTTCTAA(SEQ ID NO: 20) |
| Mut_RDRl_R | CTTGTCGTCATCGTCTTTGTAG (SEQ ID NO: 21) |
| location_OsRDR1-mcherry_F | TGCGGTACCGCGGGCATGATGGATTGGTTTATGTCTACAG(SEQ ID NO: 22) |
| location_OsRDR1-mcherry_R | ACTTCCTCTGCCCTCCTTGTACAGCTCGTCCATGC(SEQ ID NO: 23) |
| location_T2A-EGFP_F | GACGAGCTGTACAAGGAGGGCAGAGGAAGTCTTCTAA (SEQ ID NO: 24) |
| location_T2A-EGFP_R | CGACTCTAGAGTCGCTTACTTGTACAGCTCGTCCATG (SEQ ID NO: 25) |
| OsRDRp-F | TCGGCGCGCCTGCAGGTCGACATGGGGAAGACAATTCAAGTGTTT(SEQ ID NO: 26) |
| OsRDRp-R | CGACTTAAGCATTATGCGGCCGCTCAAGCTCGCTCTTTTACCTCTC(SEQ ID NO: 27) |

**Table 3. Materials used in the examples**

| Reagents or experiment resources | Source | Catalog No. |
|---|---|---|
| Antibodies | | |
| Mouse Monoclonal anti-FLAG^{®} M2-Peroxidase (HRP) | Sigma-Aldrich | Cat# A8592 |
| Rabbit Monoclonal anti-ACTB-Peroxidase (HRP) | ABclonal | Cat# AC028 |
| Rabbit Polyclonal anti-EGFR | ABclonal | Cat# A11351 |
| Rabbit Polyclonal anti-PLK1 | ABclonal | Cat# A2548 |
| Bacteria and viral strains | | |
| Trans1-T1 Phage Resistant Chemically Competent Cell | TransGen Biotech | Cat# CD501-03 |
| TransStbl3 Chemically Competent Cell | TransGen Biotech | Cat# CD521-01 |

| Chemicals, polypeptides, and recombinant proteins | | |
|---|---|---|
| DMEM | GIBCO | Cat# 11965092 |
| Opti-MEM I Reduced Serum | | |
| Medium | GIBCO | Cat# 31985070 |
| L-Glutamine | GIBCO | Cat# 25030081 |
| Sodium Pyruvate | GIBCO | Cat# 11360070 |
| Penicillin-Streptomycin | GIBCO | Cat# 15140163 |
| Trypsin-EDTA | GIBCO | Cat# 15400054 |
| DPBS | GIBCO | Cat# 14190250 |
| DMSO | Sigma-Aldrich | Cat# 67-68-5 |
| Doxycycline hyclate (Dox) | Sigma-Aldrich | Cat# D9891 |
| PMSF | Sigma-Aldrich | Cat# 329-98-6 |
| 2-Mercaptoethanol | ThermoFisher | Cat# 60-24-2 |
| Lipofectamine 3000 Transfection Reagent | ThermoFisher | Cat# L3000015 |
| Reagents or experiment resources | Source | Catalog No. |
| TRIzol Reagent | Invitrogen | Cat# 15596026 |
| D-Luciferin potassium salt | Beyotime Biotech | Cat# ST196 |
| ANTI-FLAG^{®} M2 Affinity Gel | Millipore | Cat# A2220 |

| Key commercial kits | | |
|---|---|---|
| TruSeq Stranded mRNA Sample Prep Kits | Illumina | Cat# RS-122-2101 |
| NEBNext Small RNA Library Prep Set | New England Biolabs | Cat# E7330S |
| Q5 Site-Directed Mutagenesis Kit | New England Biolabs | Cat# E0554 |
| SuperScript IV Reverse Transcriptase | Invitrogen | Cat# 18090010 |
| Platinum High Fidelity Taq DNA Polymerase | Invitrogen | Cat# 11304029 |
| RNasin Ribonuclease Inhibitors | Promega | Cat# N2518 |
| EdU Cell Proliferation Kit with Alexa Fluor 488 | Beyotime Biotech | Cat# 0071S |
| Annexin V-PE/7-AAD Apoptosis Detection Kit | Vazyme | Cat# A213-01 |

| Experiment models: cell lines | | |
|---|---|---|
| 293T | Clontech | Cat# 632617 |
| NIH/3T3 | ATCC | CRL-1658 |
| RPE-1 | ATCC | CRL-4000 |
| A549LUC | Vitalstar | N/A |
| HCT116 | ATCC | CCL-247 |
| HeLa | ATCC | CCL-2 |
| HepG2 | ATCC | HB-8065 |
| H1299 | ATCC | CRL-5803 |
| K562 | ATCC | CCL-243 |
| U2-OS | ATCC | HTB-96 |
| Mouse cell line: NIH/3T3-OsRDR1 | Lab | N/A |
| Mouse cell line: NIH/3T3-AtRDR1 | Lab | N/A |
| Human cell line: RPE-1-OsRDR1 | Lab | N/A |
| Human cell line: RPE-1-AtRDR1 | Lab | N/A |
| Human cell line: A549LUC-OsRDR1 | Lab | N/A |
| Human cell line: A549LUC-AtRDR1 | Lab | N/A |
| Human cell line: HCT116-OsRDR1 | Lab | N/A |
| Human cell line: HCT116-AtRDR1 | Lab | N/A |
| Human cell line: HeLa-OsRDR1 | Lab | N/A |
| Human cell line: HeLa-AtRDR1 | Lab | N/A |
| Human cell line: HepG2-OsRDR1 | Lab | N/A |
| Human cell line: HepG2-AtRDR1 | Lab | N/A |
| Human cell line: H1299-OsRDR1 | Lab | N/A |
| Human cell line: H1299-AtRDR1 | Lab | N/A |
| Human cell line: K562-OsRDR1 | Lab | N/A |
| Human cell line: K562-AtRDR1 | Lab | N/A |
| Human cell line: U2-OS-OsRDR1 | Lab | N/A |
| Human cell line: U2-OS-AtRDR1 | Lab | N/A |

| Experimental models: organisms/strains | | |
|---|---|---|
| Mouse: NOD.Cg-Prkdcscid Il2rgtm1 Vst/Vst | Vitalstar | N/A |

| Reagents or experiment resources | Source | Catalog No. |
|---|---|---|
| Recombinant DNA | | |
| pMD2G | Addgene | Addgen Plasmid #12259 |
| psPAX2 | Addgene | Addgen Plasmid #12260 |
| pLenti-FLAG-OsRDR1-T2A-EGFP | Lab | N/A |
| pLenti-FLAG-AtRDR1-T2A-EGFP | Lab | N/A |
| pLenti-FLAG-OsRDR1mut-T2A-EG FP | Lab | N/A |
| pLenti-FLAG-AtRDR1mut-T2A-EG FP | Lab | N/A |

| Softwares and algorithms | | |
|---|---|---|
| Bowtie | http://bowtie-bio.sourceforge. net/index.shtml | N/A |
| TopHat | https://ccb.jhu.edu/software/to phat/manual.shtml | N/A |
| Toppgene | https://toppgene.cchmc.org/ | N/A |
| Graphpad prism 7 | https://www.graphpad.com/sci entificsoftware/ prism/ | N/A |
| Perl | N/A | N/A |
| R/Bioconductor | N/A | N/A |

The content described above is only the preferable embodiments of the present disclosure, and it should be noted that several improvements and modifications can be made by the person of ordinary skill in the art without departing from the priciples of the present disclosure. These improvements and modifications should also be regarded as in the scope of the present disclosure.

## Claims

1. Use of RDR or a functional variant thereof for the preparation of a medicament for the treatment or prevention of cell proliferative diseases.

2. The use according to claim 1, wherein the cell proliferative disease is tumor, and specifically, the tumor can be benign or malignant tumor (or cancer), and preferably, the malignant tumor includes solid tumor, lymphoma or hematologic tumor, e.g., leukaemia, breast cancer, skin cancer, osteocarcinoma, liver cancer, brain cancer, laryngeal cancer, gallbladder cancer, pancreatic cancer, rectal cancer, parathyroid gland cancer, stomach cancer, bronchogenic cancer, kidney cancer, basal cell carcinoma, ulcer, and papillary squamous cell carcinoma, sarcoma, myeloma, giant cell tumor, small cell lung cancer, non-small cell lung cancer, islet cell tumor, primary brain tumor, acute or chronic lymphocytoma or granulocyte tumor, hair cell tumor, adenoma, medullary carcinoma, pheochromocytoma, mucosal nerve cell carcinoma, intestinal ganglloneuromas, proliferative corneal nerve tumor, spermatocytoma, liomyoma, cervical cancer, colorectal cancer, neuroblastoma, retinoblastoma, rhabdomyosarcoma, malignant hypercalcemia, glioblastoma multiforme, melanoma or epiermoid carcinoma.

3. The use according to claim 1 or 2, wherein the RDR is selected from the group consisting of RDR1, RDR2, RDR3, RDR4, RDR5 and RDR6, preferably, the RDR is selected from the group consisting of RDR1, RDR2 and RDR6, more preferably, the RDR is RDR1; even more preferably, the RDR is derived from a plant, such as monocotyledonous or dicotyledonous plant, preferably the plant is selected from the group consisting of *Arabidopsis thaliana,* rice, tobacco, *Populus trichocarpa,* soybean, grape, tomato, potato, barley, slender false brome, maize, sorghum, cacao, rice, petunia, cotton and cucumber.

4. The use according to any of claims 1 to 3, wherein the RDR is the nucleic acid encoding RDR protein, such as genomic DNA, cDNA, or CDS, preferably, the nucleic acid is integrated in a vector, such as a plasmid vector or a viral vector, preferably, the viral vector is selected from the group consisting of retroviral vector, lentiviral vector and adenovirus vector; or
the functional variant is a truncated RDR retaining the RDRp domain, for example, the functional variant is the RDRp domain.

5. A pharmaceutical composition for the treatment or prevention of cell proliferative diseases, comprising RDR and a pharmaceutically acceptable carrier.

6. A method for suppressing cell proliferation, comprising *in vitro* introduction of the RDR into a target cell, wherein the target cell is a normal cell, or a cell with abnormal proliferation regulation, such as a tumor cell.

7. A method for inhibiting tumor growth and/or metastasis, comprising the step of administrating a therapeutically effective amount of RDR protein, the nucleic acid encoding RDR protein, or the pharmaceutical composition according to claim 5 to a subject in need thereof.

8. A method for improving the stability or increasing the expression of miRNAs in cells, comprising the step of contacting RDR with a target cell, or introducing RDR into a target cell, preferably, the target cell is a normal cell, or a cell with abnormal proliferation regulation, such as a tumor cell or a cancer cell.

9. A method for regulating cell cycle, comprising the step of contacting RDR with a target cell, or introducing RDR into a target cell, and preferably, the target cell is a normal cell or a cell with abnormal proliferation regulation, such as a tumor cell.

10. A method for increasing the expression of low-expression miRNAs in tumor cells, comprising the step of contacting RDR with a tumor cell, or introducing RDR into a tumor cell.

11. A method for reducing the expression of cell cycle-related genes in tumor cells, comprising the step of contacting RDR with a tumor cell, or introducing RDR into a tumor cell.

12. The method according to any one of claims 6 to 11, wherein the RDR is selected from the group consisting of RDR1, RDR2, RDR3, RDR4, RDR5 and RDR6, preferably, the RDR is selected from the group consisting of RDR1, RDR2 and RDR6, more preferably, the RDR is RDR1; even more preferably, the RDR is derived from a plant, such as monocotyledonous or dicotyledonous plant, preferably the plant is selected from the group consisiting of *Arabidopsis thaliana,* rice, tobacco, *Populus trichocarpa,* soybean, grape, tomato, potato, barley, slender false brome, maize, sorghum, cacao, rice, petunia, cotton and cucumber.

13. The method according to any one of claims 6 to 12, wherein the RDR is a functional RDR protein or a nucleic acid molecule encoding RDR protein.

14. The method according to any one of claims 6 to 13, wherein the subject or cell is preferably or is preferably derived from human or animals, for example, the animals are non-human mammals, such as cattle, swine, horses, chickens, cats or dogs, etc.
